(19)
Europäisches
Patentamt
European
Patent Office
Office européen
des brevets

(11) **EP 3 114 984 A1**

(12) **EUROPEAN PATENT APPLICATION**
published in accordance with Art. 153(4) EPC

(43) Date of publication:
**11.01.2017 Bulletin 2017/02**

(51) Int Cl.:
**A61B 1/06** (2006.01)

(21) Application number: **15833909.3**

(22) Date of filing: **03.03.2015**

(86) International application number:
**PCT/JP2015/056148**

(87) International publication number:
**WO 2016/027484 (25.02.2016 Gazette 2016/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **19.08.2014 JP 2014166747**

(71) Applicant: **Olympus Corporation
Tokyo 192-8507 (JP)**

(72) Inventor: **TAKEI, Shunji
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(54) **LIGHT SOURCE DEVICE**

(57)    A light source apparatus includes a laser light source configured to emit laser light to illuminate an object, a light receiving section configured to output a light detection signal corresponding to a light quantity of at least part of the received laser light emitted from the laser light source, a calculation processing section configured to output either a calculated value obtained by integrating, for a predetermined time period, values resulting from multiplying detection values of the light detection signals by a correction coefficient or a calculated value obtained by multiplying values resulting from integrating, for a predetermined time period, detection values of the light detection signals by a correction coefficient, a determining section configured to determine whether or not the calculated value is equal to or less than a threshold, a light source control section configured to control the laser light source based on the determination result obtained by the determining section, and an adjustment coefficient calculating section configured to calculate an adjustment coefficient for adjusting the correction coefficient in response to a change in loss produced in an optical transmission line along which the laser light emitted from the laser light source is emitted to the object.

**FIG. 3**

## Description

Technical Field

The present invention relates to a light source apparatus, and more particularly, to a light source apparatus that emits laser light.

Background Art

In endoscopes in a medical field, various techniques are being proposed to reduce a diameter of an insertion portion inserted into a body cavity of a subject to alleviate a burden on the subject. A scanning type endoscope with no solid image pickup device in a part corresponding to the aforementioned insertion portion and a scanning type endoscope system constructed of the scanning type endoscope or the like are known as an example of such a technique.

More specifically, the aforementioned scanning type endoscope system is configured to swing a distal end portion of an illumination fiber that transmits light emitted from a light source, thereby two-dimensionally scan an object in a preset scanning pattern, receive return light from the object using a light receiving fiber and generate an image of the object based on the returned light received by the light receiving fiber. For example, an endoscope apparatus disclosed in Japanese Patent Application Laid-Open Publication No. 2011-255015 is known as one having a configuration similar to that of such a scanning type endoscope system.

The aforementioned scanning type endoscope system is generally configured to irradiate an object with laser light having a light quantity equal to or less than a standard value defined in a laser safety standard and scan the object. According to such a configuration, as a light quantity adjustment in consideration of loss produced in an optical transmission line formed by including an optical member such as an illumination fiber, if a light quantity adjustment is made such that the light quantity of the laser light incident on the optical transmission line from a light source is set to a light quantity greater than the aforementioned standard value to adjust the light quantity of laser light emitted to the object via the optical transmission line to the aforementioned standard value or below, there is a problem that the supply of laser light from the light source may be stopped.

On the other hand, Japanese Patent Application Laid-Open Publication No. 2011-255015 does not particularly refer to any technique or the like that can solve the aforementioned problem, that is, there are still problems to be solved associated with the aforementioned problem.

The present invention has been implemented in view of the aforementioned circumstances and it is an object of the present invention to provide a light source apparatus capable of adjusting a light quantity of laser light emitted to an object via an optical transmission line with loss produced in the optical transmission line that transmits laser light taken into consideration.

Disclosure of Invention

Means for Solving the Problems

A light source apparatus according to an aspect of the present invention includes a laser light source configured to emit laser light to illuminate an object, a light receiving section configured to receive at least part of the laser light emitted from the laser light source, and generate and output a light detection signal corresponding to a light quantity of the received laser light, a calculation processing section configured to generate and output a signal indicating either a calculated value obtained by integrating, for a predetermined time period, values resulting from multiplying detection values of the light detection signals outputted from the light receiving section by a correction coefficient or a calculated value obtained by multiplying values resulting from integrating, for a predetermined time period, detection values of the light detection signals outputted from the light receiving section by a correction coefficient, a determining section configured to determine whether or not the calculated value indicated by the signal outputted from the calculation processing section is equal to or less than a threshold, a light source control section configured to control the laser light source based on the determination result obtained by the determining section, and an adjustment coefficient calculating section configured to calculate an adjustment coefficient for adjusting the correction coefficient in response to a change in loss produced in an optical transmission line along which the laser light emitted from the laser light source is emitted to the object.

A light source apparatus according to an aspect of the present invention includes a laser light source configured to emit laser light to illuminate an object, a light receiving section configured to receive at least part of the laser light emitted from the laser light source, and generate and output a light detection signal corresponding to a light quantity of the received laser light, an integrating section configured to generate and output an integrated signal by integrating detection values of the light detection signals for a predetermined time period, a determining section configured to determine whether or not the integrated value indicated by the integrated signal outputted from the integrating section is equal to or less than a threshold, a light source control section configured to control the laser light source based on the determination result obtained by the determining section, and an adjustment coefficient calculating section configured to calculate an adjustment coefficient for adjusting the threshold in response to a change in loss produced in an optical transmission line along which the laser light emitted from the laser light source is emitted to the object.

Brief Description of the Drawings

Fig. 1 is a diagram illustrating a configuration of main parts of an endoscope system according to a first embodiment;
Fig. 2 is a diagram illustrating an example of a configuration of a light-detecting section according to the first embodiment;
Fig. 3 is a diagram illustrating an example of a configuration of a light quantity monitoring section according to the first embodiment;
Fig. 4 is a diagram illustrating a configuration of main parts of an endoscope system according to a second embodiment; and
Fig. 5 is a diagram illustrating an example of a configuration of a light quantity monitoring section according to the second embodiment.

Best Mode for Carrying Out the Invention

Hereinafter, embodiments of the present invention will be described with reference to the accompanying drawings.

(First Embodiment)

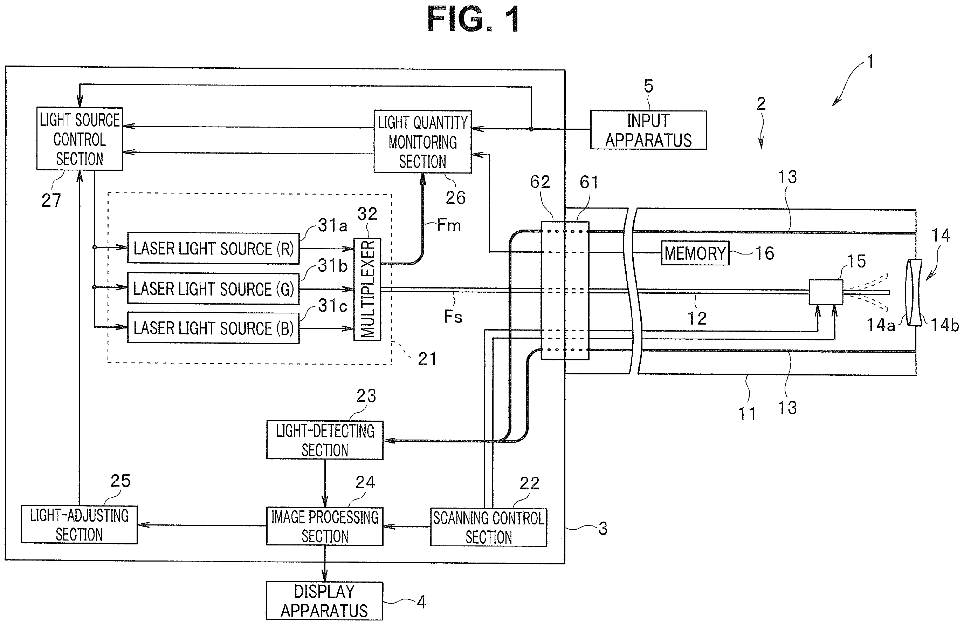

Fig. 1 to Fig. 3 relate to a first embodiment of the present invention.

As shown in Fig. 1, an endoscope system 1 is constructed of a scanning type endoscope 2 configured to be inserted into a body cavity of a subject, a main unit 3 to which the endoscope 2 can be connected, a display apparatus 4 configured to be connected to the main unit 3 and an input apparatus 5 configured to be able to input information or an instruction to the main unit 3. Note that the input apparatus 5 is not limited to the one configured as an apparatus separate from the main unit 3 as shown in Fig. 1, but may also be configured as an interface integrated with the main unit 3. Fig. 1 is a diagram illustrating a configuration of main parts of the endoscope system according to the first embodiment.

The endoscope 2 is provided with an elongated and flexible insertion portion 11 that can be inserted into a body cavity of a subject.

The insertion portion 11 is provided with a connector section 61 at its proximal end portion, configured to detachably connect the endoscope 2 to a connector receiving section 62 of the main unit 3.

Although not shown, an electric connector apparatus to electrically connect the endoscope 2 and the main unit 3 is provided inside the connector section 61 and the connector receiving section 62. An optical connector apparatus which is not shown is provided inside the connector section 61 and the connector receiving section 62 to optically connect the endoscope 2 and the main unit 3.

On the other hand, an illumination fiber 12 which is an optical fiber to transmit light supplied from the main unit 3 to a condensing optical system 14 and a light receiving fiber 13 provided with one or more optical fibers to receive return light (hereinafter, also referred to as "reflected light") from the object and transmit the return light to the main unit 3 are respectively inserted through a portion inside the insertion portion 11 from a proximal end portion to a distal end portion.

An incident end part including a light incident surface of the illumination fiber 12 is disposed inside the connector section 61. An end part including a light emitting surface of the illumination fiber 12 is disposed in the vicinity of a light incident surface of a lens 14a provided at a distal end portion of the insertion portion 11.

An incident end part including a light incident surface of the light receiving fiber 13 is fixedly disposed in the periphery of a light emitting surface of a lens 14b on a front end face of the distal end portion of the insertion portion 11. Furthermore, a light emitting end part including a light emitting surface of the light receiving fiber 13 is disposed inside the connector section 61.

That is, according to the above-described configuration, when the endoscope 2 and the main unit 3 are connected, light emitted from the main unit 3 is made incident on the light incident surface of the illumination fiber 12 provided in the connector section 61. Furthermore, according to the above-described configuration, when the endoscope 2 and the main unit 3 are connected, light made incident from the light incident surface of the light receiving fiber 13 is emitted to the main unit 3 via the connector section 61 and the connector receiving section 62.

The condensing optical system 14 is constructed of the lens 14a upon which light exiting from the light emitting surface of the illumination fiber 12 is made incident and the lens 14b configured to emit light exiting from the lens 14a to an object.

An actuator section 15 is provided at some midpoint of the illumination fiber 12 on the distal end portion side of the insertion portion 11, configured to be driven based on a drive signal supplied from a scanning control section 22 of the main unit 3 to thereby cause the light emitting end part of the illumination fiber 12 to swing.

The actuator section 15 is constructed of a first actuator provided with one or more piezoelectric elements configured to be driven based on a drive signal supplied from the scanning control section 22 of the main unit 3 to be enabled to cause the light emitting end part of the illumination fiber 12 to swing along a first direction and a second actuator provided with one or more piezoelectric elements configured to be driven based on a drive signal supplied from the scanning control section 22 of the main unit 3 to be enabled to cause the light emitting end part to swing along a second direction orthogonal to the first direction.

A memory 16 is provided inside the insertion portion 11, configured to store information including a transmittance $T_{Rf}$ of R light (which will be described later), a transmittance $T_{Gf}$ of G light (which will be described later) and a transmittance $T_{Bf}$ of B light (which will be described later) as information indicating an actual transmittance of the illumination fiber 12 when there is no bending loss.

The main unit 3 is constructed of a laser module 21, the scanning control section 22, a light-detecting section 23, an image processing section 24, a light-adjusting section 25, a light quantity monitoring section 26, and a light source control section 27.

The laser module 21 is optically connected to the connector receiving section 62 via an optical fiber Fs for illuminating light transmission provided inside the main unit 3. Furthermore, the laser module 21 is optically connected to the light quantity monitoring section 26 via an optical fiber Fm for light quantity monitoring provided inside the main unit 3. The laser module 21 is constructed of a laser light source 31a, a laser light source 31b, a laser light source 31c and a multiplexer 32.

The laser light source 31a is configured to switch on or off under the control of the light source control section 27. The laser light source 31a is also configured to generate laser light in a red wavelength band (hereinafter, also referred to as "R light") with an output value corresponding to the control of the light source control section 27.

The laser light source 31b is configured to switch on or off under the control of the light source control section 27. The laser light source 31b is also configured to generate laser light in a green wavelength band (hereinafter, also referred to as "G light") with an output value corresponding to the control of the light source control section 27.

The laser light source 31c is configured to switch on or off under the control of the light source control section 27. The laser light source 31c is also configured to generate laser light in a blue wavelength band (hereinafter, also referred to as "B light") with an output value corresponding to the control of the light source control section 27.

The multiplexer 32 is configured to emit white light obtained by multiplexing R light emitted from the laser light source 31a, G light emitted from the laser light source 31b and B light emitted from the laser light source 31c to a light incident surface of the optical fiber Fs. The multiplexer 32 is also configured to emit white light having a light quantity less than the light quantity of light emitted to the optical fiber Fs to a light incident surface of an optical fiber Fm.

That is, according to the above-described configuration, the white laser light emitted from the laser module 21 is supplied to the endoscope 2 via the optical fiber Fs and the connector receiving section 62 as illuminating light to illuminate an object. Furthermore, according to the above-described configuration, part of the white laser light emitted from the laser module 21 is made incident on the light quantity monitoring section 26 via the optical fiber Fm.

The scanning control section 22 is provided with, for example, a signal generator. The scanning control section 22 is configured to generate a drive signal to cause the light emitting end part of the illumination fiber 12 to swing in a predetermined scanning pattern such as a helical or Lissajous curve and output the generated drive signal to the actuator section 15 and the image processing section 24.

The light-detecting section 23 is configured to detect light made incident via the connector receiving section 62, generate an electric signal, amplify the generated electric signal, generate a digital signal indicating a brightness value corresponding to the amplified electric signal and output the digital signal to the image processing section 24.

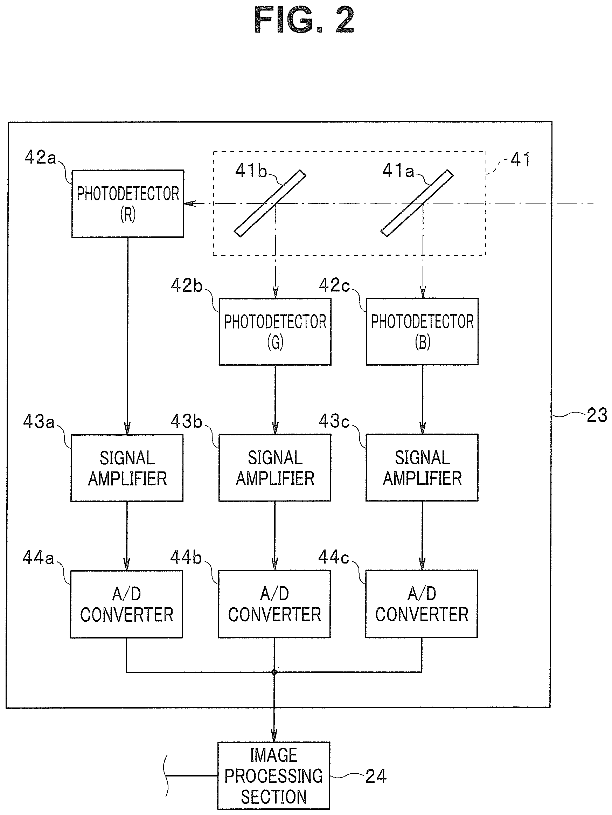

More specifically, the light-detecting section 23 is constructed of a spectroscopic optical system 41 provided with dichroic mirrors 41a and 41b, photodetectors 42a to 42c, signal amplifiers 43a to 43c, and A/D converters 44a to 44c as shown in Fig. 2. Fig. 2 is a diagram illustrating an example of a configuration of the light-detecting section according to the first embodiment.

The dichroic mirror 41a is provided with an optical characteristic so as to transmit R light and G light included in light made incident via the connector receiving section 62 toward the dichroic mirror 41b side and reflect B light included in the light to the photodetector 42c side.

The dichroic mirror 41b is provided with an optical characteristic so as to transmit R light made incident via the dichroic mirror 41a toward the photodetector 42a side and reflect G light made incident via the dichroic mirror 41a toward the photodetector 42b side.

The photodetector 42a is provided with, for example, an avalanche photodiode or a photomultiplier tube. The photodetector 42a is configured to receive R light made incident via the dichroic mirror 41b with predetermined sensitivity, and generate and output an electric signal corresponding to a light quantity of the received R light.

The photodetector 42b is provided with, for example, an avalanche photodiode or a photomultiplier tube. The photodetector 42b is configured to receive G light reflected by the dichroic mirror 41b with predetermined sensitivity, and generate and output an electric signal corresponding to a light quantity of the received G light.

The photodetector 42c is provided with, for example, an avalanche photodiode or a photomultiplier tube. The photodetector 42c is configured to receive B light reflected by the dichroic mirror 41a with predetermined sensitivity, and generate and output an electric signal corresponding to a light quantity of the received B light.

The signal amplifier 43a is configured to amplify and output the electric signal outputted from the photodetector 42a.

The signal amplifier 43b is configured to amplify and output the electric signal outputted from the photodetector 42b.

The signal amplifier 43c is configured to amplify and output the electric signal outputted from the photodetector 42c.

The A/D converter 44a is configured to convert the electric signal outputted from the signal amplifier 43a to a digital signal and output the digital signal.

The A/D converter 44b is configured to convert the electric signal outputted from the signal amplifier 43b to a digital signal and output the digital signal.

The A/D converter 44c is configured to convert the electric signal outputted from the signal amplifier 43c to a digital signal and output the digital signal.

The image processing section 24 is provided with an image processing circuit such as an AGC (auto gain control) circuit. The image processing section 24 is configured to detect a scanning pattern of an object based on a drive signal outputted from the scanning control section 22, map a brightness value indicated by a digital signal outputted from the light-detecting section 23 to a pixel at a position corresponding to the detected scanning pattern, perform an interpolation process to interpolate pixel information of each pixel which is not to be mapped and thereby generate an image of the object. The image processing section 24 is also configured to output the image of the object generated as described above to the light-adjusting section 25. The image processing section 24 applies processes such as gain adjustment to the image of the object generated as described above to thereby generate an image to be displayed and output the generated display image to the display apparatus 4.

The light-adjusting section 25 is provided with, for example, a light-adjusting circuit. Furthermore, the light-adjusting section 25 is configured to calculate an average value of brightness values of images outputted from the image processing section 24, generate a light adjustment signal to bring the difference between the calculated average value of brightness values and a predetermined brightness target value closer to 0 and output the generated light adjustment signal to the light source control section 27.

The light quantity monitoring section 26 is configured to detect light quantities of the R light, the G light and the B light included in the white light made incident via the optical fiber Fm respectively, perform a correction process to correct the detected light quantities, perform a threshold determination process based on the corrected light quantity obtained through the correction process and output the determination result obtained through the threshold determination process to the light source control section 27. The light quantity monitoring section 26 is configured to calculate an adjustment coefficient to adjust the correction coefficient used in the aforementioned correction process based on information read from the memory 16 and information inputted in response to the operation of the input apparatus 5 and output the calculated adjustment coefficient to the light source control section 27.

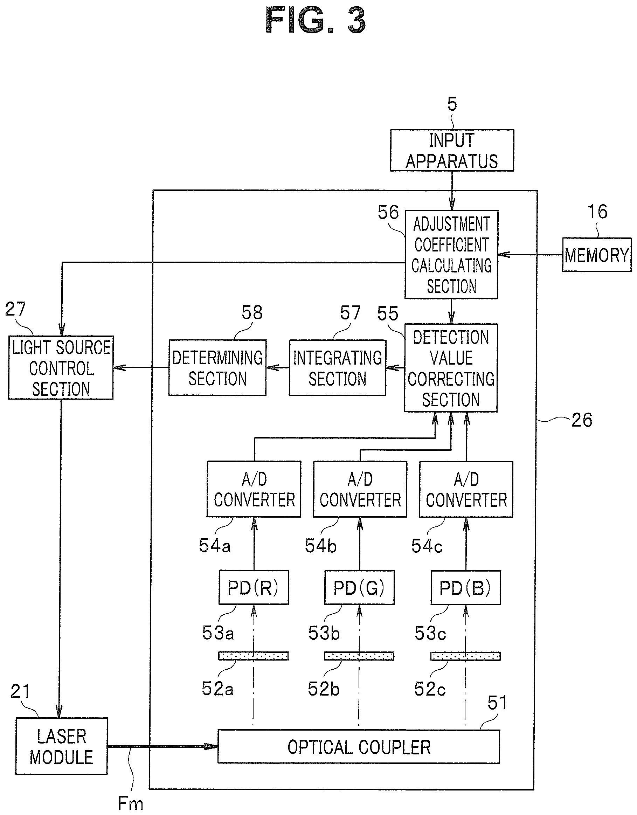

More specifically, as shown in Fig. 3, the light quantity monitoring section 26 is constructed of, for example, an optical coupler 51, optical filters 52a to 52c, photodiodes (hereinafter, abbreviated as "PD") 53a to 53c, A/D converters 54a to 54c, a detection value correcting section 55, an adjustment coefficient calculating section 56, an integrating section 57, and a determining section 58. Fig. 3 is a diagram illustrating an example of a configuration of the light quantity monitoring section according to the first embodiment.

The optical coupler 51 is configured to divide the white light emitted via the optical fiber Fm into three equal portions and emit the divided portions of the white light to the optical filters 52a, 52b and 52c respectively.

The optical filter 52a is provided with such an optical characteristic as to transmit R light included in the white light emitted from the optical coupler 51 and cut light other than the R light.

The optical filter 52b is provided with such an optical characteristic as to transmit G light included in the white light emitted from the optical coupler 51 and cut light other than the G light.

The optical filter 52c is provided with such an optical characteristic as to transmit B light included in the white light emitted from the optical coupler 51 and cut light other than the B light.

The PD 53a is configured to receive the R light emitted via the optical filter 52a, and generate and output an electric signal corresponding to a light quantity of the received R light.

The PD 53b is configured to receive the G light emitted via the optical filter 52b, and generate and output an electric signal corresponding to a light quantity of the received G light.

The PD 53c is configured to receive the B light emitted via the optical filter 52c, and generate and output an electric signal corresponding to a light quantity of the received B light.

The A/D converter 54a is configured to digitize the electric signal outputted from the PD 53a to thereby generate a light detection signal and output the light detection signal.

The A/D converter 54b is configured to digitize the electric signal outputted from the PD 53b to thereby generate a light detection signal and output the light detection signal.

The A/D converter 54c is configured to digitize the electric signal outputted from the PD 53c to thereby generate a light detection signal and output the light detection signal.

The detection value correcting section 55 is constructed of a digital signal processing circuit or the like. The detection value correcting section 55 is configured to multiply the detection value of the light detection signal outputted from the A/D converter 54a by a correction coefficient $C_R$ and output the multiplication result to the integrating section 57. The detection value correcting section 55 is configured to multiply the detection value of the light detection signal outputted from the A/D converter 54b by a correction coefficient $C_G$ and output the multiplication result to the integrating section 57. The detection value correcting section 55 is configured to multiply the detection value of the light detection signal outputted from the A/D converter 54c by a correction coefficient $C_B$ and output the multiplication result to the integrating section 57. The detection value correcting section 55 is configured to adjust each correction coefficient using an adjustment coefficient outputted from the adjustment coefficient calculating section 56 (which will be described later) to thereby calculate an adjusted correction coefficient.

Note that in the present embodiment, suppose that an initial value $C_{Rd}$ of the correction coefficient $C_R$ is calculated in advance based on a value $G_R$ which is a ratio of a light quantity of the R light emitted from the optical coupler 51 to a light quantity of the R light made incident on the optical fiber Fs, a transmittance $V_R$ of the R light passing through the main unit 3 and an ideal transmittance $T_{Ri}$ ($>T_{Rf}$) of the R light in the illumination fiber 12.

On the other hand, in the present embodiment, suppose that an initial value $C_{Gd}$ of the correction coefficient $C_G$ is calculated in advance based on a value $G_G$ which is a ratio of a light quantity of the G light emitted from the optical coupler 51 to a light quantity of the G light made incident on the optical fiber Fs, a transmittance $V_G$ of the G light passing through the main unit 3 and an ideal transmittance $T_{Gi}$ ($>T_{Gf}$) of the G light in the illumination fiber 12.

Furthermore, in the present embodiment, suppose that an initial value $C_{Bd}$ of the correction coefficient $C_B$ is calculated in advance based on a value $G_B$ which is a ratio of a light quantity of the B light emitted from the optical coupler 51 to a light quantity of the B light made incident on the optical fiber Fs, a transmittance $V_B$ of the B light passing through the main unit 3 and an ideal transmittance $T_{Bi}$ ($>T_{Bf}$) of the B light in the illumination fiber 12.

The adjustment coefficient calculating section 56 is constructed of a calculation circuit or the like. The adjustment coefficient calculating section 56 is configured to calculate an adjustment coefficient $A_R$ for adjusting the correction coefficient $C_R$, an adjustment coefficient $A_G$ for adjusting the correction coefficient $C_G$ and an adjustment coefficient $A_B$ for adjusting the correction coefficient $C_B$ based on information read from the memory 16, information inputted in accordance with operation of the input apparatus 5 and a parameter provided as a known value, and output each of the calculated adjustment coefficients to the detection value correcting section 55 and the light source control section 27. Note that the adjustment coefficient calculating section 56 is assumed to be provided with the values $G_R$, $G_G$ and $G_B$ as light quantity ratios, the transmittances $V_R$, $V_G$ and $V_B$, and the transmittances $T_{Ri}$, $T_{Gi}$ and $T_{Bi}$ as known values.

The integrating section 57 is constructed of a calculation circuit provided with an integrator or the like. The integrating section 57 is configured to integrate detection values of light detection signals outputted from the detection value correcting section 55 for a predetermined time period $\tau$ (e.g., $\tau = 0.25$ sec) to thereby generate an integrated signal and output the generated integrated signal to the determining section 58.

That is, the calculation processing section according to the present embodiment is provided with functions corresponding to those of the detection value correcting section 55 and the integrating section 57, and configured to generate a signal indicating a calculated value obtained by integrating a value resulting from multiplying the detection value of the light detection signal outputted from the A/D converter 54a by the correction coefficient $C_R$, a value resulting from multiplying the detection value of the light detection signal outputted from the A/D converter 54b by the correction coefficient $C_G$, and a value resulting from multiplying the detection value of the light detection signal outputted from the A/D converter 54c by the correction coefficient $C_B$ for the predetermined time $\tau$ and output the signal to the determining section 58.

The determining section 58 is constructed of, for example, a determination circuit. Furthermore, the determining section 58 is also configured to perform a determination process to determine whether or not the integrated value indicated by an integrated signal outputted from the integrating section 57 is equal to or less than a threshold TH and output the determination result acquired by the determination process to the light source control section 27.

Note that the aforementioned threshold TH is assumed to be set as a value less than a standard value of AEL (accessible emission limit) defined in IEC60825-1, an international standard that defines safety standards of laser products.

The light source control section 27 is provided with, for example, a CPU or a control circuit. The light source control section 27 is configured to set current values of drive currents to be supplied to the laser light sources 31 a, 31 b and 31 c based on a light adjustment signal outputted from the light-adjusting section 25.

The light source control section 27 is configured to perform control to, for example, switch on or off the laser light sources 31 a, 31b and 31c based on operation carried out by the input apparatus 5.

The light source control section 27 is configured to perform control to cause the laser light sources 31a, 31b and 31c to simultaneously emit light as control to emit white light from the laser module 21.

Based on the adjustment coefficient outputted from the adjustment coefficient calculating section 56 and the determination result outputted from the determining section 58, the light source control section 27 is configured to perform, when a determination result showing that the integrated value indicated by the integrated signal outputted from the integrating section 57 is equal to or less than the threshold TH is obtained, control to emit white light provided with a light quantity corresponding to the adjustment coefficient from the laser module 21 on the laser light sources 31 a, 31b and 31 c.

Based on the determination result outputted from the determining section 58, when a determination result is obtained which indicates that the integrated value indicated by the integrated signal outputted from the integrating section 57 is greater than the threshold TH, the light source control section 27 is configured to perform control to stop emission of white light from the laser module 21 on the laser light sources 31a, 31b and 31c.

Next, operation of the present embodiment will be described. Note that a case will be described hereinafter as an example where a process is started, as a starting point, from a state in which correction coefficients stored in the detection value correcting section 55 are initial values $C_{Rd}$, $C_{Gd}$ and $C_{Bd}$, that is, a state in which the adjustment coefficient calculating section 56 has not calculated adjustment coefficients.

The user such as an operator makes connections of respective components of the endoscope system 1, turns on power and then turns on an illumination switch (not shown) of the input apparatus 5 to thereby instruct the main unit 3 to start to supply illuminating light.

Upon detecting that the illumination switch of the input apparatus 5 is turned on, the light source control section 27 performs control to cause the laser light sources 31 a, 31 b and 31 c to simultaneously emit R light, G light and B light. In accordance with such control, white light provided with a predetermined light quantity is made incident on the optical fiber Fs and white light provided with a light quantity less than the predetermined light quantity is made incident on the optical fiber Fm.

When the endoscope 2 is connected to the main unit 3, the adjustment coefficient calculating section 56 reads information stored in the memory 16, calculates adjustment coefficients $A_{R1}$, $A_{G1}$ and $A_{B1}$ based on the read information and outputs the calculated adjustment coefficients $A_{R1}$, $A_{G1}$ and $A_{B1}$ to the detection value correcting section 55 and the light source control section 27.

More specifically, the adjustment coefficient calculating section 56 divides the transmittance $_{Rf}$ by the transmittance $T_{Ri}$ to thereby calculate an adjustment coefficient $A_{R1}$ (= $T_{Rf}/T_{Ri}$) for adjusting the correction coefficient $C_{Rd}$. Furthermore, the adjustment coefficient calculating section 56 divides the transmittance $T_{Gf}$ by the transmittance $T_{Gi}$ based on the information read from the memory 16 to thereby calculate an adjustment coefficient $A_{G1}$ (= $T_{Gf}/T_{Gi}$) for adjusting the correction coefficient $C_{Gd}$. The adjustment coefficient calculating section 56 divides the transmittance $T_B$ by the transmittance $T_{Bi}$ based on the information read from the memory 16 to thereby calculate an adjustment coefficient $A_{B1}$ (= $T_{Bf}/T_{Bi}$) for adjusting the correction coefficient $C_{Bd}$.

That is, the adjustment coefficient calculating section 56 calculates the adjustment coefficients $A_{R1}$, $A_{G1}$ and $A_{B1}$ for adjusting the correction coefficients $C_{Rd}$, $C_{Gd}$ and $C_{Bd}$ in response to a change in loss produced in the optical transmission line along which laser light emitted from the laser module 21 is emitted to the object via the illumination fiber 12.

The detection value correcting section 55 multiplies the correction coefficient $C_{Rd}$ by the adjustment coefficient $A_{R1}$ outputted from the adjustment coefficient calculating section 56 to thereby acquire an adjusted correction coefficient $C_{R1}$.
The detection value correcting section 55 multiplies the correction coefficient $C_{Gd}$ by the adjustment coefficient $A_{G1}$ outputted from the adjustment coefficient calculating section 56 to thereby acquire an adjusted correction coefficient $C_{G1}$. The detection value correcting section 55 multiplies the correction coefficient $C_{Bd}$ by the adjustment coefficient $A_{B1}$ outputted from the adjustment coefficient calculating section 56 to thereby acquire an adjusted correction coefficient $C_{B1}$.

After that, the detection value correcting section 55 multiplies the detection value of the light detection signal outputted from the A/D converter 54a by the correction coefficient $C_{R1}$ and outputs the multiplication result to the integrating section 57, multiplies the detection value of the light detection signal outputted from the A/D converter 54b by the correction coefficient $C_{G1}$ and outputs the multiplication result to the integrating section 57, and multiplies the detection value of the light detection signal outputted from the A/D converter 54c by the correction coefficient $C_{B1}$ and outputs the multiplication result to the integrating section 57.

Here, when the incident light quantity of R light incident on the PD 53a is assumed to be $I_R$, the incident light quantity of G light incident on the PD 53b is assumed to be $I_G$, the incident light quantity of B light incident on the PD 53c is assumed to be $I_B$, the emitted light quantity of R light included in white light emitted from the emission end face of the illumination fiber 12 is assumed to be $P_R$, the emitted light quantity of G light included in the white light is assumed to be $P_G$, the emitted light quantity of B light included in the white light is assumed to be $P_B$, the transmittance of R light in the illumination fiber 12 is assumed to be $T_R$, the transmittance of G light in the illumination fiber 12 is assumed to be $T_G$, and the transmittance of B light in the illumination fiber 12 is assumed to be $T_B$, relationships as shown in following equations (1) to (3) hold true.

$$I_R = \left( G_R / V_R \cdot T_R \right) \cdot P_R \quad \cdots \quad (1)$$

$$I_G = \left( G_G / V_G \cdot T_G \right) \cdot P_G \quad \cdots \quad (2)$$

$$I_B = \left(G_B / V_B \cdot T_B\right) \cdot P_B \quad \cdots \quad (3)$$

That is, the relational expressions shown in above equations (1) to (3) can be used as substitutes that hold true with the transmittances $T_{Rf}$, $T_{Gf}$ and $T_{Bf}$ corresponding to the illumination fiber 12 instead of the ideal transmittances $T_{Ri}$, $T_{Gi}$ and $T_{Bi}$ accompanying the connection of the endoscope 2.

Therefore, a process for adjusting the correction coefficients $C_{Rd}$, $C_{Gd}$ and $C_{Bd}$ to the correction coefficients $C_{R1}$, $C_{G1}$ and $C_{B1}$ is carried out as described above, and the determining section 58 thereby makes a threshold determination adapted to the emitted light quantity emitted from the emission end face of the illumination fiber 12, and as a result, it is possible to adjust the light quantity of laser light emitted via the illumination fiber 12 while considering loss produced when there is no bending loss in the illumination fiber 12.

Next, processes carried out when adjusting the correction coefficients $C_{R1}$, $C_{G1}$ and $C_{B1}$ in response to a replacement of the laser module 21 will be described.

The user such as an operator replaces the laser module 21 and turns on power to each section of the endoscope system 1, then operates the input apparatus 5 to input information indicating the fact that the laser module 21 is replaced.

Based on the operation of the input apparatus 5, the light source control section 27 performs, upon detecting that the laser module 21 is replaced, control to cause the laser light sources 31 a, 31b and 3 c to simultaneously emit R light, G light and B light so that the incident light quantities $I_R$, $I_G$ and $I_B$ are set to predetermined values respectively.

On the other hand, using a measuring device such as an optical power meter, the user measures an emitted light quantity $P_{R1}$ of R light included in white light emitted from the endoscope 2 after inputting information indicating that the laser module 21 is replaced, an emitted light quantity $P_{G1}$ of G light included in the white light and an emitted light quantity $P_{B1}$ of B light included in the white light respectively. By operating the input apparatus 5, the user inputs the measured values of the emitted light quantities $P_{R1}$, $P_{G1}$ and $P_{B1}$ measured as described above respectively.

The adjustment coefficient calculating section 56 calculates adjustment coefficients $A_{R2}$, $A_{G2}$ and $A_{B2}$ based on the information read from the memory 16 and the measured values of the emitted light quantities $P_{R1}$, $P_{G1}$ and $P_{B1}$ inputted in response to the operation of the input apparatus 5 and outputs the calculated adjustment coefficients $A_{R2}$, $A_{G2}$ and $A_{B2}$ to the detection value correcting section 55 and the light source control section 27.

More specifically, the adjustment coefficient calculating section 56 applies the incident light quantities $I_R$, $I_G$ and $I_B$ which are known values, the emitted light quantities $P_{R1}$, $P_{G1}$, and $P_{B1}$ and the transmittances $T_{Rf}$, $T_{Gf}$ and $T_{Bf}$ which are inputted in response to the operation of the input apparatus 5 to following equations (4) to (6) respectively and thereby calculates values of $G_{R1}N_{R1}$, $G_{G1}N_{G1}$ and $G_{B1}N_{B1}$.

$$I_R = \left(G_{R1} / V_{R1} \cdot T_{Rf}\right) \cdot P_{R1} \quad \cdots \quad (4)$$

$$I_G = \left(G_{G1} / V_{G1} \cdot T_{Gf}\right) \cdot P_{G1} \quad \cdots \quad (5)$$

$$I_B = \left(G_{B1} / V_{B1} \cdot T_{Bf}\right) \cdot P_{B1} \quad \cdots \quad (6)$$

$G_{R1}$ in above equation (4) represents a ratio of a light quantity of R light emitted from the optical coupler 51 to a light quantity of R light incident on the optical fiber Fs after the replacement of the laser module 21. $V_{R1}$ in above equation (4) represents a transmittance of R light that passes through the main unit 3 after the replacement of the laser module 21.

$G_{G1}$ in above equation (5) represents a ratio of a light quantity of G light emitted from the optical coupler 51 to a light quantity of G light incident on the optical fiber Fs after the replacement of the laser module 21. $V_{G1}$ in above equation (5) represents a transmittance of G light that passes through the main unit 3 after the replacement of the laser module 21.

$G_{B1}$ in above equation (6) represents a ratio of a light quantity of B light emitted from the optical coupler 51 to a light quantity of B light incident on the optical fiber Fs after the replacement of the laser module 21. $V_{B1}$ in above equation (6) represents a transmittance of B light that passes through the main unit 3 after the replacement of the laser module 21.

The adjustment coefficient calculating section 56 applies the values of $G_{R1}/V_{R1}$, $G_{G1}/V_{G1}$ and $G_{B1}/V_{B1}$ calculated as described above, the values of the light quantity ratios of $G_R$, $G_G$ and $G_B$, and the transmittances $V_R$, $V_G$ and $V_B$ to following equations (7) to (9) respectively and thereby calculates an adjustment coefficient $A_{R2}$ corresponding to $P_R/P_{R1}$, an adjustment coefficient $A_{G2}$ corresponding to $P_G/P_{G1}$ and an adjustment coefficient $A_{B2}$ corresponding to $P_B/P_{B1}$ respectively.

$$A_{R2} = \left(G_R / V_R\right) / \left(G_{R1} / V_{R1}\right) \quad \cdots \quad (7)$$

$$A_{G2} = \left(G_G / V_G\right) / \left(G_{G1} / V_{G1}\right) \quad \cdots \quad (8)$$

$$A_{B2} = \left(G_B / V_B\right) / \left(G_{B1} / V_{B1}\right) \quad \cdots \quad (9)$$

That is, the adjustment coefficient calculating section 56 calculates the adjustment coefficients $A_{R2}$, $A_{G2}$ and $A_{B2}$ to adjust the correction coefficients $C_{R1}$, $C_{G1}$ and $C_{B1}$ in response to a change in emitted light quantities of laser light emitted to the object via the optical transmission line including the illumination fiber 12.

The detection value correcting section 55 multiplies the correction coefficient $C_{R1}$ by the adjustment coefficient $A_{R2}$ outputted from the adjustment coefficient calculating section 56 to thereby acquire an adjusted correction coefficient $C_{R2}$.

The detection value correcting section 55 multiplies the correction coefficient $C_{G1}$ by the adjustment coefficient $A_{G2}$ outputted from the adjustment coefficient calculating section 56 to thereby acquire an adjusted correction coefficient $C_{G2}$. The detection value correcting section 55 multiplies the correction coefficient $C_{B1}$ by the adjustment coefficient $A_{B2}$ outputted from the adjustment coefficient calculating section 56 to thereby acquire an adjusted correction coefficient $C_{B2}$.

After that, the detection value correcting section 55 multiplies the detection value of the light detection signal outputted from the A/D converter 54a by the correction coefficient $C_{R2}$ and outputs the multiplication result to the integrating section 57, multiplies the detection value of the light detection signal outputted from the A/D converter 54b by the correction coefficient $C_{G2}$ and outputs the multiplication result to the integrating section 57, and multiplies the detection value of the light detection signal outputted from the A/D converter 54c by the correction coefficient $C_{B2}$ and outputs the multiplication result to the integrating section 57.

Therefore, a process for adjusting the correction coefficients $C_{R1}$, $C_{G1}$ and $C_{B1}$ to the correction coefficients $C_{R2}$, $C_{G2}$ and $C_{B2}$ is carried out as described above, and the determining section 58 thereby makes a threshold determination adapted to the emitted light quantity emitted from the emission end face of the illumination fiber 12, and as a result, it is possible to adjust the light quantity of laser light emitted via the illumination fiber 12 while considering loss produced when there is no bending loss in the illumination fiber 12 in response to the replaced laser module 21.

Next, a process carried out when adjusting the correction coefficients $C_{R1}$, $C_{G1}$ and $C_{B1}$ in accordance with the bending state of the insertion portion 11 inserted through the guide member will be described. Note that for simplicity, a case where the guide member is a tubular member formed of a metal or the like, that is, a case where the insertion portion 11 inserted through the guide member is bent according to a minimum bending radius minR and a maximum bending angle max$\theta$ of the guide member will be described hereinafter as an example.

The user such as an operator turns on power to each section of the endoscope system 1, and operates, for example, the input apparatus 5 to thereby input the values of the minimum bending radius minR and the maximum bending angle max$\theta$ of the guide member through which the insertion portion 11 is inserted.

Based on the information read from the memory 16, the value of the minimum bending radius minR inputted in response to the operation of the input apparatus 5 and the value of the maximum bending angle max$\theta$ inputted in response to the operation of the input apparatus 5, the adjustment coefficient calculating section 56 calculates a transmittance $T_{Rb}$ of R light, a transmittance $T_{Gb}$ of G light and a transmittance $T_{Bb}$ of B light respectively as information indicating the actual transmittance of the illumination fiber 12 when there is bending loss.

Note that according to the present embodiment, the input apparatus 5 may receive the type of the guide member though which the insertion portion 11 is inserted and the adjustment coefficient calculating section 56 may perform a process of extracting the values of the minimum bending radius minR and the maximum bending angle max$\theta$ in accordance with the inputted type of the guide member from a database or the like.

Based on the information read from the memory 16 and the transmittances $T_{Rb}$, $T_{Gb}$ and $T_{Bb}$ calculated as described above, the adjustment coefficient calculating section 56 calculates adjustment coefficients $A_{R3}$, $A_{G3}$ and $A_{B3}$ and outputs the calculated adjustment coefficients $A_{R3}$, $A_{G3}$ and $A_{B3}$ to the detection value correcting section 55 and the light source control section 27.

More specifically, the adjustment coefficient calculating section 56 divides the transmittance $T_{Rb}$ by the transmittance $T_{Rf}$ and thereby calculates the adjustment coefficient $A_{R3}$ (= $T_{Rb}/T_{Rf}$) for adjusting the correction coefficient $C_{R1}$. The adjustment coefficient calculating section 56 divides the transmittance $T_{Gb}$ by the transmittance $T_{Gf}$ and thereby calculates the adjustment coefficient $A_{G3}$ (= $T_{Gb}/T_{Gf}$) for adjusting the correction coefficient $C_{G1}$ based on the information read from the memory 16. Furthermore, the adjustment coefficient calculating section 56 divides the transmittance $T_{Bb}$ by the transmittance $T_{Bf}$ and thereby calculates the adjustment coefficient $A_{B3}$ (= $T_{Bb}/T_{Bf}$) for adjusting the correction coefficient $C_{B1}$ based on the information read from the memory 16.

That is, the adjustment coefficient calculating section 56 calculates the adjustment coefficients $A_{R3}$, $A_{G3}$ and $A_{B3}$ to adjust the correction coefficients $C_{R1}$, $C_{G1}$ and $C_{B1}$ in response to a change in loss produced in the optical transmission line along which laser light emitted from the laser module 21 is made incident on the object via the illumination fiber 12.

Note that according to the present embodiment, when calculating, for example, the adjustment coefficients $A_{R3}$, $A_{G3}$ and $A_{B3}$, white balance coefficients may also be used to keep white balance with the insertion portion 11 inserted through the guide member.

The detection value correcting section 55 multiplies the correction coefficient $C_{R1}$ by the adjustment coefficient $A_{R3}$ outputted from the adjustment coefficient calculating section 56 to thereby acquire the adjusted correction coefficient $C_{R3}$. The detection value correcting section 55 multiplies the correction coefficient $C_{G1}$ by the adjustment coefficient $A_{G3}$ outputted from the adjustment coefficient calculating section 56 to thereby acquire the adjusted correction coefficient $C_{G3}$. The detection value correcting section 55 multiplies the correction coefficient $C_{B1}$ by the adjustment coefficient $A_{B3}$ outputted from the adjustment coefficient calculating section 56 to thereby acquire the adjusted correction coefficient $C_{B3}$.

After that, the detection value correcting section 55 multiplies the detection value of the light detection signal outputted from the A/D converter 54a by the correction coefficient $C_{R3}$ and outputs the multiplication result to the integrating section 57, multiplies the detection value of the light detection signal outputted from the A/D converter 54b by the correction coefficient $C_{G3}$ and outputs the multiplication result to the integrating section 57, and multiplies the detection value of the light detection signal outputted from the A/D converter 54c by the correction coefficient $C_{B3}$ and outputs the multiplication result to the integrating section 57.

The relational expressions shown in above-described equations (1) to (3) are used as substitutes that hold true with the actual transmittances $T_{Rb}$, $T_{Gb}$ and $T_{Bb}$ of the illumination fiber 12 when there is bending loss instead of the actual transmittances $T_{Rf}$, $T_{Gf}$ and $T_{Bf}$ of the illumination fiber 12 when there is no bending loss.

Therefore, a process for adjusting the correction coefficients $C_{R1}$, $C_{G1}$ and $C_{B1}$ to the correction coefficients $C_{R3}$, $C_{G3}$ and $C_{B3}$ is carried out as described above, and the determining section 58 thereby makes a threshold determination adapted to the emitted light quantity emitted from the emission end face of the illumination fiber 12, and as a result, it is possible to adjust the light quantity of laser light emitted via the illumination fiber 12 while considering loss produced in accordance with bending loss of the illumination fiber 12.

(Second Embodiment)

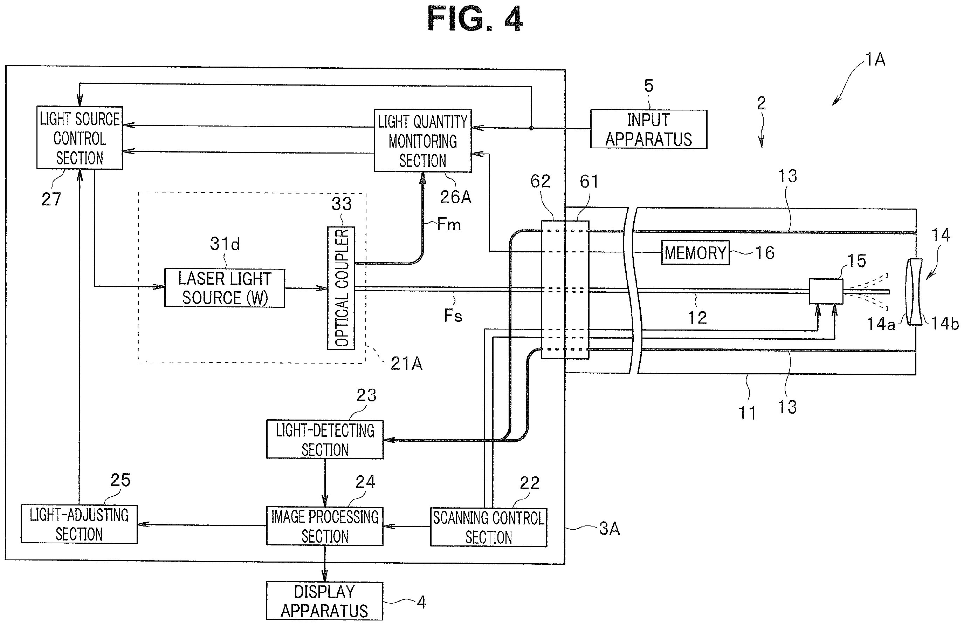

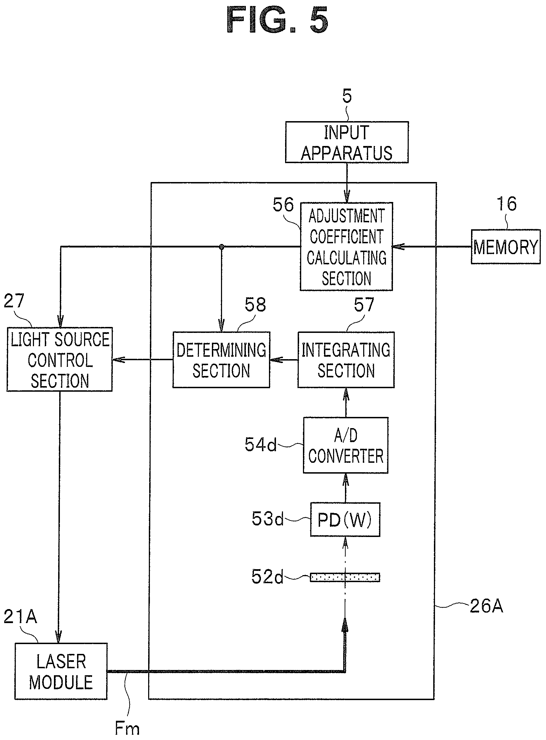

Fig. 4 and Fig. 5 relate to a second embodiment of the present invention.

Note that in the present embodiment, detailed description of parts including components similar to those of the first embodiment is omitted and parts having components different from those in the first embodiment will be mainly described.

As shown, for example, in Fig. 4, an endoscope system 1A is constructed of the endoscope 2, a main unit 3A to which the endoscope 2 can be connected, the display apparatus 4 connected to the main unit 3A and the input apparatus 5 configured to be able to input information or give an instruction to the main unit 3A. Fig. 4 is a diagram illustrating a configuration of main parts of an endoscope system according to the second embodiment.

The memory 16 of the present embodiment stores information including a transmittance $T_{wf}$ of W light (which will be described later) as information indicating an actual transmittance of the illumination fiber 12 when there is no bending loss.

The main unit 3A is constructed of a laser module 21 A, the scanning control section 22, the light-detecting section 23, the image processing section 24, the light-adjusting section 25, a light quantity monitoring section 26A, and the light source control section 27.

The laser module 21 A is optically connected to the connector receiving section 62 via the optical fiber Fs for illuminating light transmission provided inside the main unit 3A. Furthermore, the laser module 21A is optically connected to the light quantity monitoring section 26A via the optical fiber Fm for light quantity monitoring provided inside the main unit 3A. The laser module 21A is constructed of a laser light source 31d and an optical coupler 33.

The laser light source 31 d is configured to switch on or off in accordance with the control of the light source control section 27. The laser light source 31d is configured to generate white laser light (hereinafter, also referred to as "W light") with an output value corresponding to the control of the light source control section 27 when the laser light source 31d is on.

The optical coupler 33 is configured to divide the W light emitted from the laser light source 31d, thereby emit the W light having a light quantity L1 to the light incident surface of the optical fiber Fs and emit the W light having a light quantity L2 which is less than the light quantity L1 to the light incident surface of the optical fiber Fm.

That is, according to the above-described configuration, the W light emitted from the laser module 21A is supplied to the endoscope 2 via the optical fiber Fs and the connector receiving section 62 as illuminating light to illuminate an object. Moreover, according to the above-described configuration, part of the W light emitted from the laser module 21A is made incident on the light quantity monitoring section 26A via the optical fiber Fm.

The light quantity monitoring section 26A detects the light quantity of the W light incident via the optical fiber Fm, performs a threshold determination process based on the detected light quantity and output the determination result obtained by the threshold determination process to the light source control section 27. The light quantity monitoring section 26A is also configured to calculate an adjustment coefficient for adjusting a threshold used in the aforementioned threshold determination process based on the information read from the memory 16 and the information inputted in response to the operation of the input apparatus 5 and output the calculated adjustment coefficient to the light source control section 27.

More specifically, the light quantity monitoring section 26A is constructed, as shown, for example, in Fig. 5, of an optical filter 52d, and a PD 53d, an A/D converter 54d, an adjustment coefficient calculating section 56, an integrating section 57, and a determining section 58. Fig. 5 is a diagram illustrating an example of a configuration of the light quantity monitoring section according to the second embodiment.

The optical filter 52d is formed so as to have a transmittance corresponding to a reciprocal of light-receiving sensitivity of the PD 53d in a wavelength band of W light emitted via the optical fiber Fm.

The PD 53d is configured to receive the W light emitted via the optical filter 52d with predetermined light-receiving sensitivity, and generate and output an electric signal corresponding to the light quantity of the received W light.

The A/D converter 54d is configured to digitize the electric signal outputted from the PD 53d to thereby generate and output a light detection signal.

The adjustment coefficient calculating section 56 is configured to calculate an adjustment coefficient $A_w$ for adjusting a threshold TH used in a determination process by the determining section 58 based on the information read from the memory 16, information inputted in response to the operation of the input apparatus 5 and a parameter provided as a known value, and output the calculated adjustment coefficient $A_w$ to the determining section 58 and the light source control section 27. Note that the adjustment coefficient calculating section 56 of the present embodiment is assumed to include a value $G_w$ (= L1/L2) of a ratio of a light quantity L1 of W light incident on the optical fiber Fm to a light quantity L2 of W light incident on the optical fiber Fs, a transmittance $V_w$ of the W light passing through the main unit 3 and an ideal transmittance $T_{wi}$ ($>T_w$) of the W light in the illumination fiber 12 as known values.

The integrating section 57 is configured to integrate detection values of light detection signals outputted from the A/D converter 54d for a predetermined time period $\tau$ (e.g., $\tau$ = 0.25 sec), thereby generate an integrated signal and output the generated integrated signal to the determining section 58.

The determining section 58 is configured to adjust a threshold TH using the adjustment coefficient $A_w$ outputted from the adjustment coefficient calculating section 56 to thereby calculate an adjusted threshold THA. The determining section 58 is configured to perform a determination process to determine whether or not the integrated value indicated by an integrated signal outputted from the integrating section 57 is equal to or less than the threshold THA and output the determination result acquired by the determination process to the light source control section 27.

The light source control section 27 is configured to perform control to cause the laser module 21A to emit the W light having a light quantity corresponding to the adjustment coefficient on the laser light source 31d based on the adjustment coefficient outputted from the adjustment coefficient calculating section 56 and the determination result outputted from the determining section 58 when a determination result is obtained indicating that the integrated value indicated by the integrated signal outputted from the integrating section 57 is equal to or less than the threshold THA.

The light source control section 27 is configured to perform control to cause the laser module 21A to stop emission of the W light on the laser light source 31d based on the determination result outputted from the determining section 58 when a determination result is obtained indicating that the integrated value indicated by the integrated signal outputted from the integrating section 57 is greater than the threshold THA.

Next, operation of the present embodiment will be described. Note that a case will be described hereinafter where a process is started as a starting point from a state in which a threshold stored in the determining section 58 is TH, that is, a state in which the adjustment coefficient calculating section 56 has not calculated the adjustment coefficient.

The user such as an operator connects each section of the endoscope system 1A, turns on power and then turns on, for example, an illumination switch (not shown) of the input apparatus 5 and thereby instructs the main unit 3A to start supply of illuminating light.

Upon detecting that the illumination switch of the input apparatus 5 is turned on, the light source control section 27 performs control to turn on the laser light source 31d. In response to such control, the W light having the light quantity L1 is made incident on the optical fiber Fs and the W light having the light quantity L2 is made incident on the optical fiber Fm.

When the endoscope 2 is connected to the main unit 3A, the adjustment coefficient calculating section 56 reads information stored in the memory 16, calculates an adjustment coefficient $A_{w1}$ based on the read information and outputs the calculated adjustment coefficient $A_{w1}$ to the determining section 58 and the light source control section 27.

More specifically, the adjustment coefficient calculating section 56 divides the transmittance $T_{wi}$ by the transmittance $T_{Wf}$ to calculate the adjustment coefficient $A_{w1}$ (= $T_{Wi}/T_{Wf}$) for adjusting the threshold TH.

That is, the adjustment coefficient calculating section 56 calculates the adjustment coefficient $A_{W1}$ for adjusting the threshold TH in response to a change in loss produced in the optical transmission line along which laser light emitted from the laser module 21 is emitted to the object via the illumination fiber 12.

The determining section 58 multiplies the threshold TH by the adjustment coefficient $A_{w1}$ outputted from the adjustment coefficient calculating section 56 to thereby acquire an adjusted threshold TH1. The determining section 58 performs a determination process to determine whether or not the integrated value indicated by the integrated signal outputted from the integrating section 57 is equal to or less than the threshold TH1 and outputs the determination result acquired by the determination process to the light source control section 27.

Here, when the incident light quantity of the W light made incident on the PD 53d is assumed to be $I_W$, the emitted light quantity of white light emitted from the emission end face of the illumination fiber 12 is assumed to be $P_W$ and the transmittance of the W light in the illumination fiber 12 is assumed to be $T_W$, a relationship as shown in following equation (10) holds true.

$$I_W = \left(G_W/V_W \cdot T_W\right) \cdot P_W \quad \cdot \cdot \cdot \quad (1\,0)$$

That is, the relational expression shown in above-described equation (10) is used as a substitute assumed to hold true with the transmittance $T_{Wf}$ corresponding to the illumination fiber 12 instead of the ideal transmittance $T_{Wi}$ accompanying the connection of the endoscope 2.

Therefore, the process for adjusting the threshold TH to the threshold TH1 is carried out as described above, and the determining section 58 thereby makes a threshold determination adapted to the emitted light quantity emitted from the emission end face of the illumination fiber 12, and as a result, it is possible to adjust the light quantity of laser light emitted via the illumination fiber 12 while considering loss produced when there is no bending loss in the illumination fiber 12.

Next, a process carried out when adjusting the threshold TH1 in response to a replacement of the laser module 21A will be described.

The user such as an operator replaces the laser module 21 A, turns on power to each section of the endoscope system 1A, then operates, for example, the input apparatus 5 and thereby inputs information indicating that the laser module 21A is replaced.

Upon detecting that the laser module 21A is replaced based on the operation of the input apparatus 5, the light source control section 27 controls the light source 31 d so as to emit the W light such that the incident light quantity $I_W$ becomes a predetermined value.

On the other hand, the user measures the emitted light quantity $P_{W1}$ of white light emitted from the endoscope 2 after inputting the information indicating that the laser module 21A is replaced using a measuring device such as an optical power meter. The user then operates the input apparatus 5 to thereby input measured values of the emitted light quantity $P_{W1}$ measured as described above.

The adjustment coefficient calculating section 56 calculates an adjustment coefficient $A_{W2}$ based on the information read from the memory 16 and the measured value of the emitted light quantity $P_{W1}$ inputted in response to the operation of the input apparatus 5 and outputs the calculated adjustment coefficient $A_{W2}$ to the determining section 58 and the light source control section 27.

More specifically, the adjustment coefficient calculating section 56 applies the incident light quantity $I_W$ which is a known value, the emitted light quantity $P_{W1}$ inputted in response to the operation of the input apparatus 5 and the transmittance $T_{Wf}$ to following equation (11) to thereby calculate a value of the $G_{W1}/V_{W1}$.

$$I_W = \left(G_{W1}/V_{W1} \cdot T_{Wf}\right) \cdot P_{W1} \quad \cdot \cdot \cdot \quad (1\,1)$$

$G_{W1}$ in above-described equation (11) indicates a ratio of the light quantity L1 of the W light incident on the optical fiber Fm to the light quantity L2 of the W light incident on the optical fiber Fs after replacing the laser module 21. $V_{W1}$ in above-described equation (11) indicates a transmittance of the W light that passes through the main unit 3 after replacing the laser module 21.

The adjustment coefficient calculating section 56 applies the value of $G_{W1}/V_{W1}$, the value $G_W$ of the light quantity ratio and the transmittance $V_W$ calculated as described above to following equation (12) to thereby calculate the adjustment coefficient $A_{W2}$. That is, the adjustment coefficient $A_{W2}$ in following equation (12) indicates a value of the ratio corresponding to $P_{W1}/P_W$.

$$A_{W2} = \left(G_{W1}/V_{W1}\right)/\left(G_W/V_W\right) \quad \cdot \cdot \cdot \quad (1\,2)$$

That is, the adjustment coefficient calculating section 56 calculates the adjustment coefficient $A_{W2}$ for adjusting the threshold TH1 according to a change in loss produced in the optical transmission line along which laser light emitted from the laser module 21 is emitted to the object via the illumination fiber 12.

The determining section 58 multiplies the threshold TH1 by the adjustment coefficient $A_{W2}$ outputted from the adjustment coefficient calculating section 56 to thereby acquire an adjusted threshold TH2. The determining section 58 performs a determination process to determine whether or not the integrated value indicated by the integrated signal outputted from the integrating section 57 is equal to or less than the threshold TH2 and outputs the determination result acquired by the determination process to the light source control section 27.

Therefore, the process for adjusting the threshold TH1 to the threshold TH2 is carried out as described above, and the determining section 58 thereby makes a threshold determination adapted to the emitted light quantity emitted from the emission end face of the illumination fiber 12, and as a result, it is possible to adjust the light quantity of laser light emitted via the illumination fiber 12 according to the laser module 21A after the replacement while considering loss produced when there is no bending loss in the illumination fiber 12.

Next, a process carried out when adjusting the threshold TH1 according to the bending state of the insertion portion 11 inserted through the guide member will be described. Note that, for simplicity, a case will be described hereinafter where the guide member is a tubular member formed of a metal or the like, that is, a case where the insertion portion 11 inserted through the guide member is bent according to the minimum bending radius minR and the maximum bending angle max$\theta$ of the guide member as an example.

The user such as an operator turns on power to each section of the endoscope system 1A, then operates, for example, the input apparatus 5 to thereby input the values of the minimum bending radius minR and the maximum bending angle max$\theta$.

The adjustment coefficient calculating section 56 calculates a transmittance $T_{Wb}$ of the W light as information indicating the actual transmittance of the illumination fiber 12 when there is bending loss based on the information read from the memory 16, the value of the minimum bending radius minR inputted in response to the operation of the input apparatus 5 and the value of the maximum bending angle max$\theta$ inputted in response to the operation of the input apparatus 5.

The adjustment coefficient calculating section 56 calculates an adjustment coefficient $A_{W3}$ based on the information read from the memory 16 and the transmittance $T_{Wb}$ calculated as described above and outputs the calculated adjustment coefficient $A_{W3}$ to the determining section 58 and the light source control section 27.

More specifically, the adjustment coefficient calculating section 56 divides the transmittance $T_{Wf}$ by the transmittance $T_{Wb}$ to thereby calculate the adjustment coefficient $A_{W3}$ (= $T_{Wf}/T_{Wb}$) to adjust the threshold TH1.

That is, the adjustment coefficient calculating section 56 calculates the adjustment coefficient $A_{W3}$ to adjust the threshold TH1 according to a change in loss produced in the optical transmission line along which laser light emitted from the laser module 21 is emitted to the object via the illumination fiber 12.

The determining section 58 multiplies the threshold TH1 by the adjustment coefficient $A_{W3}$ outputted from the adjustment coefficient calculating section 56 to thereby acquire an adjusted threshold TH3. The determining section 58 performs a determination process to determine whether or not the integrated value indicated by the integrated signal outputted from the integrating section 57 is equal to or less than the threshold TH3 and outputs the determination result acquired by the determination process to the light source control section 27.

Here, the relational expression shown in above-described equation (10) is used as a substitute assumed to hold true with the actual transmittance $T_{Wb}$ of the illumination fiber 12 when there is bending loss instead of the actual transmittance $T_{Wf}$ of the illumination fiber 12 when there is no bending loss accompanying the bending of the endoscope 2.

Therefore, the process for adjusting the threshold TH1 to the threshold TH3 is carried out as described above, and the determining section 58 thereby makes a threshold determination adapted to the emitted light quantity emitted from the emission end face of the illumination fiber 12, and as a result, it is possible to adjust the light quantity of laser light emitted via the illumination fiber 12 while considering loss produced according to bending loss of the illumination fiber 12.

Note that the present embodiment is not limited to the case where a determination process is performed based on the threshold TH3 obtained by multiplying the threshold TH1 by the adjustment coefficient $A_{W3}$, but, for example, the integrated value indicated by the integrated signal outputted from the integrating section 57 may be multiplied by $1/A_{W3}$ which is a reciprocal of the adjustment coefficient $A_{W3}$ as a correction coefficient and a determination process may be performed for determining whether or not the calculated value obtained by the calculation is equal to or less than the threshold TH1.

The present invention is not limited to the aforementioned embodiments but it goes without saying that various modifications or applications can be made without departing from the spirit and scope of the invention.

The present application claims priority based on Japanese Patent Application No. 2014-166747, filed in Japan on August 19, 2014, the disclosure of which is incorporated in the specification, claims and drawings of the present application by reference.

## Claims

1.   A light source apparatus comprising:

a laser light source configured to emit laser light to illuminate an object;
a light receiving section configured to receive at least part of the laser light emitted from the laser light source, and generate and output a light detection signal corresponding to a light quantity of the received laser light;
a calculation processing section configured to generate and output a signal indicating either a calculated value obtained by integrating, for a predetermined time period, values resulting from multiplying detection values of the light detection signals outputted from the light receiving section by a correction coefficient or a calculated value obtained by multiplying values resulting from integrating, for a predetermined time period, detection values of the light detection signals outputted from the light receiving section by a correction coefficient;
a determining section configured to determine whether or not the calculated value indicated by the signal outputted from the calculation processing section is equal to or less than a threshold;
a light source control section configured to control the laser light source based on the determination result obtained by the determining section; and
an adjustment coefficient calculating section configured to calculate an adjustment coefficient for adjusting the correction coefficient in response to a change in loss produced in the optical transmission line along which the laser light emitted from the laser light source is emitted to the object.

2. A light source apparatus comprising:

a laser light source configured to emit laser light to illuminate an object;
a light receiving section configured to receive at least part of the laser light emitted from the laser light source, and generate and output a light detection signal corresponding to a light quantity of the received laser light;
an integrating section configured to generate and output an integrated signal by integrating detection values of the light detection signals for a predetermined time period;
a determining section configured to determine whether or not the integrated value indicated by the integrated signal outputted from the integrating section is equal to or less than a threshold;
a light source control section configured to control the laser light source based on the determination result obtained by the determining section; and
an adjustment coefficient calculating section configured to calculate an adjustment coefficient for adjusting the threshold in response to a change in loss produced in an optical transmission line along which the laser light emitted from the laser light source is emitted to the object.

3. The light source apparatus according to claim 1, wherein the adjustment coefficient calculating section calculates the adjustment coefficient based on an ideal transmittance of the optical fiber included in the optical transmission line and an actual transmittance of the optical fiber.

4. The light source apparatus according to claim 1, wherein the adjustment coefficient calculating section calculates the adjustment coefficient based on a transmittance when there is no bending loss in an optical fiber included in the optical transmission line and a transmittance when there is bending loss in the optical fiber.

5. The light source apparatus according to claim 1, wherein the adjustment coefficient calculating section calculates the adjustment coefficient based on a change in an emitted light quantity of laser light emitted to the object via the optical transmission line.

6. The light source apparatus according to claim 2, wherein the adjustment coefficient calculating section calculates the adjustment coefficient based on an ideal transmittance of an optical fiber included in the optical transmission line and an actual transmittance of the optical fiber.

7. The light source apparatus according to claim 2, wherein the adjustment coefficient calculating section calculates the adjustment coefficient based on a transmittance when there is no bending loss in the optical fiber included in the optical transmission line and a transmittance when there is bending loss in the optical fiber.

8. The light source apparatus according to claim 2, wherein the adjustment coefficient calculating section calculates the adjustment coefficient based on a change in the emitted light quantity of the laser light emitted to the object via the optical transmission line.

# FIG. 1

LIGHT SOURCE
CONTROL
SECTION — 27

LIGHT QUANTITY
MONITORING
SECTION — 26

INPUT
APPARATUS — 5

1

2

62 · 61

13

LASER LIGHT SOURCE (R) — 31a

LASER LIGHT SOURCE (G) — 31b

LASER LIGHT SOURCE (B) — 31c

MULTIPLEXER — 32

Fm

MEMORY — 16

15

14

21

Fs

12

14a    14b

11 · 13

LIGHT-DETECTING
SECTION — 23

LIGHT-ADJUSTING
SECTION — 25

IMAGE PROCESSING
SECTION — 24

SCANNING CONTROL
SECTION — 22

3

DISPLAY
APPARATUS — 4

EP 3 114 984 A1

# FIG. 2

# FIG. 3

# FIG. 4

# FIG. 5

## INTERNATIONAL SEARCH REPORT

| International application No. |
|---|
| PCT/JP2015/056148 |

### A. CLASSIFICATION OF SUBJECT MATTER
*A61B1/06*(2006.01)i

According to International Patent Classification (IPC) or to both national classification and IPC

### B. FIELDS SEARCHED

Minimum documentation searched (classification system followed by classification symbols)
A61B1/06

Documentation searched other than minimum documentation to the extent that such documents are included in the fields searched
Jitsuyo Shinan Koho        1922-1996   Jitsuyo Shinan Toroku Koho   1996-2015
Kokai Jitsuyo Shinan Koho   1971-2015   Toroku Jitsuyo Shinan Koho   1994-2015

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2011-255015 A (Hoya Corp.), 22 December 2011 (22.12.2011), paragraphs [0009] to [0010], [0019] to [0020], [0029], [0031] to [0038]; fig. 1 to 6 (Family: none) | 1-8 |
| A | JP 2011-19706 A (Hoya Corp.), 03 February 2011 (03.02.2011), paragraphs [0006] to [0010] (Family: none) | 1-8 |

☐ Further documents are listed in the continuation of Box C.       ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 08 May 2015 (08.05.15) | 19 May 2015 (19.05.15) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011255015 A

- JP 2014166747 A